# NEUE EUROPÄISCHE PATENTSCHRIFT

(11) **EP 1 202 832 B2**
(45) Veröffentlichungstag und Bekanntmachung des Hinweises auf die Entscheidung über den Einspruch: **03.01.2007**
(45) Hinweis auf die Patenterteilung: 23.06.2004
(21) Anmeldenummer: 00954155.8
(22) Anmeldetag: 11.08.2000
(51) Int. Cl.: B23K 9/32

(54) **DATENANZEIGE AM SCHWEISSSCHIRM**
DATA DISPLAY ON A WELDING SCREEN
AFFICHAGE DE DONNEES SUR UN MASQUE DE SOUDAGE

(30) Priorität: 13.08.1999 AT 139999
(43) Veröffentlichungstag der Anmeldung: 08.05.2002
(73) Patentinhaber: Fronius International GmbH, 4643 Pettenbach (AT)
(72) Erfinder: FRIEDL, Helmut, A-4621 Sipbachzell (AT); NIEDEREDER, Franz, A-4652 Fischlham (AT); HACKL, Heinrich, A-4551 Ried/Traunkreis (AT)
(74) Vertreter: Secklehner, Günter
(86) Internationale Anmeldenummer: PCT/AT2000/000219
(87) Internationale Veröffentlichungsnummer: WO 2001/012376

(56) Entgegenhaltungen:
- EP-A- 0 091 514
- EP-B- 0 642 776
- DE-A- 4 128 291
- DE-A- 19 621 664
- US-A- 4 882 769
- US-A- 5 317 643
- US-A- 5 377 032
- PATENT ABSTRACTS OF JAPAN vol. 1997, no. 08, 29. August 1997 (1997-08-29) & JP 09 098395 A (YOKOGAWA ELECTRIC CORP), 8. April 1997 (1997-04-08)

## Beschreibung

Die Erfindung betrifft eine Schweißanlage mit einem Schweißgerät und einer Schutzvorrichtung, insbesondere einen Schweißschinn oder eine Schutzabdeckung bei Schweißautomaten, sowie ein Steuerverfahren für eine Schweißanlage mit einem Schweißgerät und einer Schutzvorrichtung und ein Verfahren zum Anzeigen von Daten oder Bildern, insbesondere von Schweißparametern, an einem Schutzschirm, wie sie in den Ansprüchen 1, 5, 15 und 20 beschrieben ist.

Es ist bereits eine Schweißanlage mit einem Schweißgerät und einer Schutzvorrichtung, insbesondere einem Schutzschirm für einen Benutzer, bekannt, bei dem die Schutzvorrichtung ein elektrisch regelbares Schutzvisier mit einer Ansteuervorrichtung aufweist und das Schweißgerät zumindest durch eine Stromquelle, einen Schweißbrenner und eine Steuervorrichtung gebildet ist. Bei dieser Art von Schweißanlage wird eine selbständige bzw. automatische Verdunkelung des Schutzvisiers durchgeführt, wobei dazu in der Schutzvorrichtung ein lichtempfindlicher Sensor angeordnet ist. Wird dabei bei einem Schweißprozeß ein Lichtbogen gezündet, so wird durch die hohe Lichtintensität des Lichtbogens diese über den Sensor erkannt, worauf von dem Sensor ein Signal an die Ansteuervorrichtung übersandt wird. Daraufhin wird von der Ansteuervorrichtung eine Verdunkelung des Schutzvisiers durch Anlegen von Strom und Spannung durchgeführt.

Nachteilig ist hierbei, daß für die Einleitung der Verdunkelung des Schutzvisiers zuerst der Lichtbogen gezündet werden muß, so daß der Benutzer über einen kurzen Augenblickdervollen Intensität des Lichtbogens ausgesetzt ist und es zu Folgeschäden an den Augen des Benutzers bzw. Schweißers kommen kann.

Weiters ist aus der DE 296 02 946 U1 eine Schweißanlage bzw. eine Schutzvorrichtung, insbesondere ein Schweißschirm, für den Schutz vor einem Schweißprozeß bzw. einem Lichtbogen bekannt, bei dem die Schutzvorrichtung durch einen Handgriff mit einem Schutzschild und ein im Schutzschild angeordnetes, durchsichtiges Schutzvisier angeordnet ist. Die Schutzvorrichtung ist mit einer elektronisch regelbaren Schweißstromquelle bzw. einem Schweißgerät über ein Kabel bzw. eine Leitung oder drahtlos verbunden. Zur Einstetlung der Stromstärke des Schweißstroms und/ oder des Schweißdrahtvorschubs ist ein Signalgeber in die mit dem Handgriff versehenen Schutzvorrichtung integriert, wobei der Signalgeber von einem Schweißer ohne Lösen der Hand von dem Handgriff betätigbar ist.

Nachteilig ist hierbei, daß zum Ablesen der Einstellwerte der Benutzer die Schutzvorrichtung aufgrund des sehr stark verdunkelten Schutzvisiers von seinem Gesicht nehmen muß um nämlich die Anzeigeelemente am Schweißgerät bzw. an der Schweißstromquelle erkennen zu können, oder daß der Benutzer ohne Kenntnisse der Einstellwerte eine Verstellung über den Sensorgeber vornimmt, wodurch es zu Fehlschweißungen bzw. zu qualitätsverminderte Schweißnähte, aufgrund von falsch eingestellten Schweißparametern, kommen kann.

Aus der JP 09 098395 A ist eine Schutzvorrichtung für die Augen bekannt, wobe die Schutzvorrichtung für die Befestigung an einem Benutzer, insbesondere an dessen Kopf, ein Traggestell und im Bereich des Gesichtes des Benutzers ein Schutzschild aufweist, das mit dem Traggestell einer Bilderzeugungsvorrichtung verbunden ist, welche eine Daten- und/oder Bildanzeige erzeugt.

Weiters sind Schutzvorrichtungen bekannt, bei denen zum Befestigen der Schutzvorrichtung am Schweißer die Schutzvorrichtung anstelle des Handgriffes ein Traggestell aufweist und somit eine schwenkbare Befestigung am Kopf des Benutzers bzw. Schweißers möglich ist.

Nachteilig ist hier wiederum, daß zum Ablesen der Schweißeinstellungen der Benutzer bzw. Schweißer die Schutzvorrichtung aufgrund des hohen Verdunkelungsgrades des Schutzvisiers die Schutzvorrichtung entfernen muß.

Ein weiterer wesentlicher Nachteil der vorgenannten Ausführungen zum Stand derTechnik liegt darin, daß bei einem Einsatz eines elektronisch, regelbaren Schutzvisiers der Benutzer bzw. Schweißer zum Ablesen der Schweißeinstellungen ohne die Schutzvorrichtung von seinem Gesicht zu lösen den Schweißprozeß unterbrechen bzw. beenden muß, um die Verdunkelung des Schutzvisiers aufzuheben.

Aus der DE 40 37 879 A1 ist eine Anzeigevorrichtung bzw. Bilderzeugungsvorrichtung für einen Helm gezeigt, bei der die Anzeigevorrichtung eine Anzeigeeinheit, die an der Innenseite des Helms angeordnet ist, für die Anzeige von Information bzw. Daten aufweist. Das Anzeigelicht von der Anzeigeeinheit wird durch eine Innenseite des am Helm angeordneten, durchsichtigen Visiers an eine Beobachtungsposition reflektiert, so daß ein Benützer ein virtuelles Bild vom Inneren des Helms beobachten bzw. erkennen kann.

Nachteilig ist hierbei, daß ein derartiger Helm zum Schutz vor Schweißprozessen nicht anwendbar oder einsetzbar ist.

Der Erfindung liegt die Aufgabe zugrunde, eine Schweißanlage mit einem Schweißgerät und einer Schutzvorrichtung, insbesondere einen Schweißschirm oder eine Schutzabdeckungen bei Schweißautomaten, sowie ein Steuerverfahren für eine Schweißanlage mit einem Schweißgerät und einer Schutzvorrichtung und ein Verfahren zum Anzeigen von Daten oder Bildern, insbesondere von Schweißparametern, an einem Schutzschirm zu schaffen, bei der dem Benutzer bzw. Schweißer eine uneingeschränkte Bewegungsfreiheit ermöglicht wird.

Die Aufgabe der Erfindung wird durch die Merkmale im Kennzeichenteil des Anspruches 1 gelöst. Vorteilhaft ist hierbei, daß vor dem Zünden des Lichtbogens durch Aussendung eines Signals die Ansteuervorrichtung bereits den Verdunkelungsvorgang des Schutzvisiers eingeleitet bzw. beendet hat und somit der Schweißer, insbesondere die Augen des Schweißers vor dem Lichtbogen geschützt sind. Ein weiterer Vorteil liegt darin, daß bei dem Einsatz einer drahtlosen Übertragung des Signals keine Bewegungseinschränkungen für den Schweißer entstehen.

Es ist auch eine Ausgestaltung nach Anspruch 2 von Vorteil, da durch die Koppelung der Schutzvorrichtung mit der Schweißanlage bzw. dem Schweißgerät eine einfache Anpassung des Verdunkelungsvorganges an die unterschiedlichen Schweißverfahren möglich ist, wodurch in einfacher Form der Verdunkelungsgrad von der Steuervorrichtung festgelegt werden kann und somit der Schaltungsaufbau der Ansteuervorrichtung für das Schutzvisier in der Schutzvorrichtung vereinfacht werden kann, so daß eine erhebliche Gewichtseinsparung erreicht wird.

Vorteilhaft ist auch eine Weiterbildung nach Anspruch 3, da dadurch auch ein Einsatz der Schutzvorrichtung ohne der Kopplung an die Schweißanlage bzw. an das Schweißgerät möglich ist.

Von Vorteil ist auch eine Ausbildung nach Anspruch 4, da dadurch nach dem Beenden des Schweißprozesses, insbesondere nach dem Beenden des Lichtbogens, das Schutzvisier deaktiviert bzw. die Verdunkelung des Schutzvisiers aufgehoben wird.

Vorteilhaft bei einer Ausbildung nach Anspruch 5 ist, daß der Schweißer auch bei verdunkeltem Schutzvisier die wichtigsten Schweißparameter ablesen kann und somit bei einer Verstellung eines Schweißparameters während des Schweißprozesses dieser nicht unterbrochen werden muß, um den neu eingestellten Wert ablesen zu können. Ein weiterer wesentlicher Vorteil liegt darin, daß durch die Erzeugung einer virtuellen Daten- und/oder Bildanzeige auch unterschiedliche Verfahrenszustände des Schweißprozesses, wie z.B. einem Kurzschluß, stabiler Lichtbogen, Zündung des Lichtbogens usw., dem Schweißer angezeigt werden können und somit auch während des verdunkelten Schutzvisiers eine exakte Überprüfung bzw. Kontrolle des Schweißprozesses möglich ist und somit die Schweißqualität verbessert werden kann.

Es sind auch die Ausgestaltungen nach den Ansprüchen 6 bis 13 von Vorteil, da durch den Einsatz einer Videokamera bzw. eines optischen Aufnahmegerätes ein virtuelles Bild in Echtzeit erzeugt wird und somit dem Schweißer ein Bild der Umgebung und/oder des Schweißprozesses gezeigt wird, so daß je nach der Kopfbewegung der Schweißer Einfluß auf das Bild nehmen kann, wodurch ein ständiges Abnehmen der Schutzvorrichtung unnötig ist.

Weiters umfaßt die Erfindung ein Steuerverfahren für eine Schweißanlage mit einem Schweißgerät und einer Schutzvorrichtung, wie es im Oberbegriff des Anspruches 14 beschrieben ist.

Die Aufgabe der Erfindung wird durch die Maßnahmen im Kennzeichenteil des Anspruches 14 gelöst. Vorteilhaft ist hierbei, daß in einfacher Weise ein Datentransfer bzw. ein Datenaustausch zwischen der Schweißanlage und der Schutzvorrichtung durchgeführt werden kann und somit die Dunkelschaltung des Schutzvisiers in Abhängigkeit des Schweißprozesses gesteuert werden kann.

Es sind auch die Maßnahmen nach Ansprüchen 15 bis 18 von Vorteil, da dadurch sichergestellt wird, daß vor dem Zünden des Lichtbogens bereits das Schutzvisier verdunkelt wurde und nach dem Beenden des Schweißprozesses die Verdunkelung des Schutzvisiers aufgehoben wird.

Vorteilhaft ist bei einer Ausführung nach Anspruch 19, daß durch die Bildung von einer virtuellen Daten- und/oder Bildanzeige der Schweißer jederzeit bestimmte Schweißparameter auch bei verdunkeltem Schutzvisier ablesen kann. Ein weiterer Vorteil liegt darin, daß dadurch bei Schweißanlagen, bei denen das Schweißgerät weiter entfernt von der Schweißstelle abgestellt ist, der Schweißer die Einstellungen über die Schutzvorrichtung ablesen kann und somit keine Probeschweißungen mehr notwendig sind bzw. daß der Benutzer nicht mehr zu dem Schweißgerät hingehen muß.

Schließlich sind auch die Maßnahmen nach den Ansprüchen 20 bis 25 von Vorteil, da dadurch dem Schweißer bzw. Benutzer die Möglichkeit geschaffen wird, sich die für ihn wichtigsten Schweißparameter an der Schutzvorrichtung anzeigen zu lassen.

Die Erfindung wird im nachfolgenden anhand der in den Zeichnungen dargestellten Ausführungsbeispiele näher erläutert.

Es zeigen:
- Fig. 1: ein Schaubild einer Schweißmaschine bzw. eines Schweißgerätes mit einer erfindungsgemäßen Schutzvorrichtung, in vereinfachter, schematischer Darstellung;
- Fig. 2: ein weiteres Schaubild der Schweißanlage mit der erfindungsgemäßen Schutzvorrichtung, in vereinfachter, schematischer Darstellung;
- Fig. 3: ein Schaubild aus Sicht eines Schweißers durch die erfindungsgemäße Schützvorrichtung, in vereinfachter, schematischer Darstellung.

Einführend wird festgehalten, daß gleiche Teile der einzelnen Ausführungsbeispiele mit gleichen Bezugszeichen versehen werden. Die in den einzelnen Ausführungsbeispielen angegebenen Lageangaben sind bei einer Lageänderung sinngemäß auf die neue Lage zu übertragen.

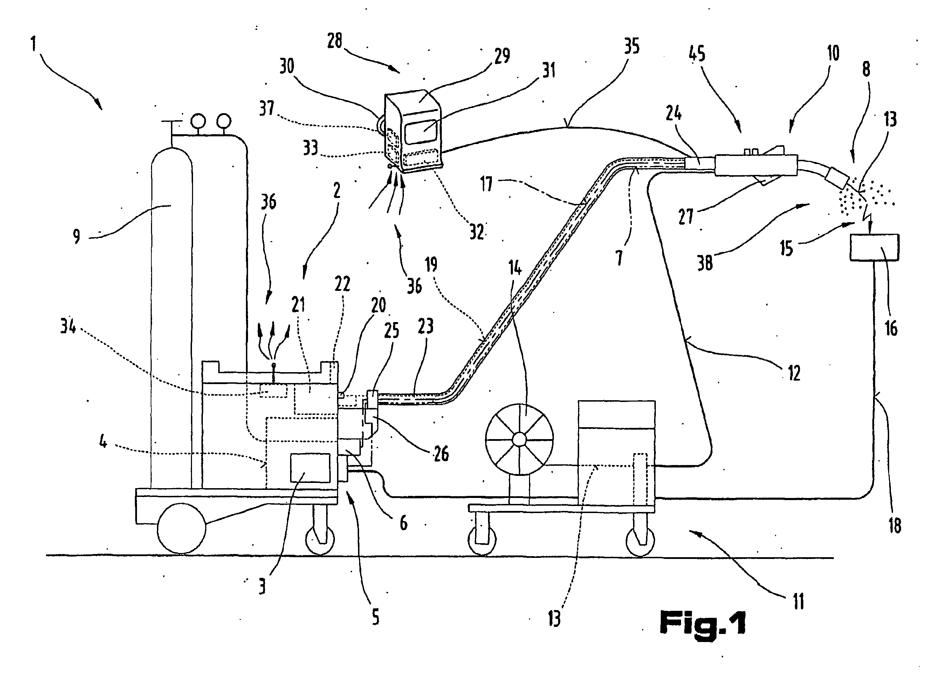

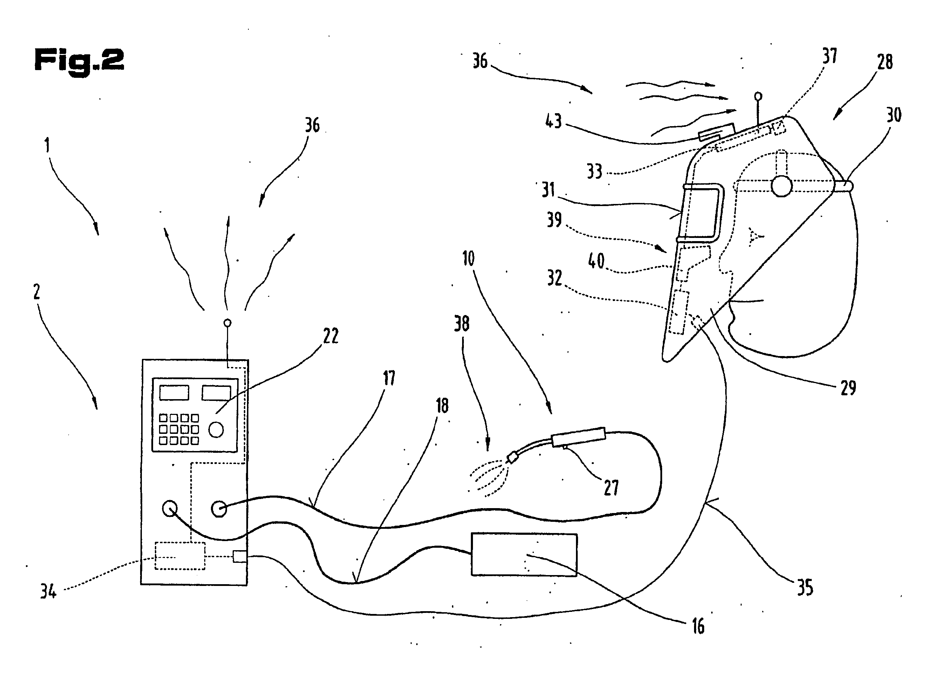

In den Fig. 1, 2 und 3 ist eine Schweißanlage bzw. ein Schweißgerät 1 für verschiedenste Schweißverfahren wie z.B. MIG/MAG-Schweißen bzw. TIG-Schweißen oder Elektroden-Schweißverfahren mit oder ohne einer Schutzgasatmosphäre gezeigt. Selbstverständlich ist es möglich, daß die erfindungsgemäße Lösung bei einer Stromquelle bzw. einer Schweißstromquelle eingesetzt werden kann.

Das Schweißgerät 1 umfaßt eine Stromquelle 2 mit einem Leistungsteil 3, einer Steuervorrichtung 4 und einem dem Leistungsteil 3 bzw. der Steuervorrichtung 4 zugeordnetem Umschaltglied 5. Das Umschaltglied 5 bzw. die Steuervorrichtung 4 ist mit einem Steuerventil 6 verbunden, welches in einer Versorgungsleitung 7 für ein Gas 8, insbesondere ein Schutzgas, wie beispielsweise CO₂, Helium oder Argon und dgl., zwischen einem Gasspeicher 9 und einem Schweißbrenner 10 angeordnet ist.

Zudem kann über die Steuervorrichtung 4 noch ein Drahtvorschubgerät 11, welches beispielsweise für das MIG/MAG-Schweißen üblich ist, angesteuert werden, wobei über eine Versorgungsleitung 12 ein Schweißdraht 13 von einer Vorratstrommel 14 in den Bereich des Schweißbrenners 10 zugeführt wird. Selbstverständlich ist es möglich, daß das Drahtvorschubgerät 11, wie es aus dem Stand der Technik bekannt ist, im Schweißgerät 1, insbesondere im Grundgehäuse, integriert ist und nicht, wie in Fig. 1 dargestellt, als Zusatzgerät ausgebildet ist.

Der Strom zum Aufbauen eines Lichtbogens 15 zwischen dem Schweißdraht 13 und einem Werkstück 16 wird über eine Schweißleitung 17 vom Leistungsteil 3 dem Schweißbrenner 10 bzw. dem Schweißdraht 13 zugeführt, wobei das zu verschweißende Werkstück 16 über eine weitere Schweißleitung 18 ebenfalls mit dem Schweißgerät 1, insbesondere mit dem Leistungsteil 3, verbunden ist und somit über dem Lichtbogen 15 ein Stromkreis aufgebaut werden kann.

Zum Kühlen des Schweißbrenners 10 kann über einen Kühlkreislauf 19 der Schweißbrenner 10 unter Zwischenschaltung eines Strömungswächters 20 mit einem Flüssigkeitsbehälter, insbesondere einem Wasserbehälter 21, verbunden werden, wodurch bei der Inbetriebnahme des Schweißbrenners 10 der Kühlkreislauf 19, insbesondere eine für die im Wasserbehälter 21 angeordnete Flüssigkeit verwendete Flüssigkeitspumpe, gestartet wird und somit eine Kühlung des Schweißbrenners 10 bzw. des Schweißdrahtes bewirkt werden kann.

Das Schweißgerät 1 weist weiters eine Ein- und/oder Ausgabevorrichtung 22 auf, über die die unterschiedlichsten Schweißparameter bzw. Betriebsarten des Schweißgerätes 1 eingestellt werden können. Dabei werden die über die Ein- und/oder Ausgabevorrichtung 22 eingestellten Schweißparameter an die Steuervorrichtung 4 weitergeleitet und von dieser werden anschließend die einzelnen Komponenten der Schweißanlage bzw. des Schweißgerätes 1 angesteuert.

Weiters ist in dem dargestellten Ausführungsbeispiel der Schweißbrenner 10 über ein Schlauchpaket 23 mit dem Schweißgerät 1 bzw. der Schweißanlage verbunden. In dem Schlauchpaket 23 sind die einzelnen Leitungen vom Schweißgerät 1 zum Schweißbrenner 10 angeordnet. Das Schlauchpaket 23 wird über eine zum Stand der Technik zählende Verbindungsvorrichtung 24 mit dem Schweißbrenner 10 verbunden, wogegen die einzelnen Leitungen im Schlauchpaket 23 mit den einzelnen Kontakten des Schweißgerätes 1 über Anschlußbuchsen bzw. Steckverbindungen verbunden sind. Damit eine entsprechende Zugentlastung des Schlauchpaketes 23 gewährleistet ist, ist das Schlauchpaket 23 über eine Zugentlastungsvorrichtung 25 mit einem Gehäuse 26, insbesondere mit dem Grundgehäuse des Schweißgerätes 1, verbunden.

Damit vom Benutzer bzw. Schweißer ein Schweißprozeß gestartet werden kann, ist am Schweißbrenner 10 ein Startschalter 27 angeordnet, d. h., daß durch Aktivieren des Startschalters 27 vom Schweißer ein Signal erzeugt wird, welches über zumindest eine Leitung an die Steuervorrichtung 4 weitergeleitet wird, so daß die Steuervorrichtung 4 erkennen kann, daß ein Schweißprozeß und somit eine Zündung des Lichtbogens gestartet werden soll und somit alle notwendigen Verfahrensschritte, wie das Starten eines Gasvorlaufes, die Zündung des Lichtbogens, die Aktivierung des Drahtvorschubgerätes 11 usw., von der Steuervorrichtung 4 eingeleitet werden. Diese einzelnen Verfahrensschritte sind bereits aus dem Stand der Technik bekannt und werden somit nicht mehr näher beschrieben, Selbstverständlich ist es möglich, daß der Startschalter 27 an der Ein- und/oder Ausgabevorrichtung 22 oder zusätzlich an dieser angeordnet sein kann.

Damit der Schweißer, insbesondere dessen Augen, vor dem Lichtbogen 15, insbesondere vor der hohen Lichtintensität des Lichtbogens 15, und/oder den entstehenden Schweißspritzern geschützt wird, wird vom Schweißer eine entsprechende Schutzvorrichtung 28, insbesondere ein Schutzschirm 29, verwendet, der, wie bereits aus dem Stand der Technik bekannt, am Kopf des Schweißers über ein Traggesteil 30 befestigt wird kann oder über einen Handgriff vor das Gesicht, insbesondere vor die Augen, gehalten wird.

Bei der dargestellten Schweißanlage mit dem Schweißgerät 1 und der Schutzvorrichtung 28, insbesondere dem Schutzschirm 29 für den Benutzer bzw. den Schweißer, weist die Schutzvorrichtung 28 ein elektrisch regelbares Schutzvisier 31 mit einer Ansteuervorrichtung 32 auf, d.h., daß das Schutzvisier 31 durch Anlegen von Energie, insbesondere von Strom und Spannung, eine Verdunkelung des Schutzvisiers 31 hervorgerufen wird und somit der Benutzer vor der Lichtintensität des Lichtbogens 15 geschützt wird, wie dies bereits bekannt ist.

Die Steuervorrichtung 4 leitet dabei ein Steuerverfahren für eine Schweißanlage mit dem Schweißgerät 1 und der Schutzvorrichtung 28, insbesondere dem Schutzschirm 29 für den Benutzer, bei dem durch Betätigen des Startschalters 27 beispielsweise an dem Schweißbrenner 10 oder dem Schweißgerät 1 ein Startsignal an die Steuervorrichtung 4 des Schweißgerätes oder einer Stromquelle zum Aktivieren eines Schweißprozesses erzeugt wird, wobei bei der Aktivierung des Startschalters 27 ein Signal bzw. Funksignal oder ein Startbefehl über eine Sende- und/oder Empfangsvorrichtung 33 und 34 an die Ansteuervorrichtung 32 der Schutzvorrichtung 28 übersandt wird, worauf von der Ansteuervorrichtung 32 ein elektrisch steuer- und/oder regelbares Schutzvisier durch Anlegen von Energie aktiviert wird und anschließend der Schweißprozeß, insbesondere die Zündung des Lichtbogens, bevorzugt nach Ablauf einer voreinstellbaren Vorlaufzeit und/oder einer Gasvorlaufzeit, in der die Verdunkelung des Schutzvisiers erfolgt, gestartet wird. Dabei ist es auch möglich, daß erst nach einer Rückantwort von der Ansteuervorrichtung, welche durch einfaches Aussenden von einem Rückantwortsignal über die Sende- und/oder Empfangsvorrichtung 33 erfolgt, eine Zündung des Lichtbogens von der Steuervorrichtung 4 eingeleitet wird, wodurch immer sichergestellt ist, daß bei der Zündung das Schutzvisier bereits dunkel geschaltet ist.

Bei den aus dem Stand derTechnik bekannten Schutzvorrichtungen 28 oder Steuerverfahren ist hierzu ein lichtempfindlicher Sensor mit der Ansteuervorrichtung 32 verbunden, so daß aufgrund der Zündung des Lichtbogens 15 der Sensor durch die hohe Lichtintensität aktiviert wird und ein Signal an die Ansteuervorrichtung 32 übersendet, welche anschließend eine entsprechende Energie an das Schutzvisier 31 anlegt.

Die erfindungsgemäße Schutzvorrichtung 28 weist eine Sende- und/oder Empfangsvorrichtung 33 auf, welche mit der Ansteuervorrichtung 32 verbunden ist. Die Sende- und/oder Empfangsvorrichtung 33 ist mit der Schweißanlage oder dem Schweißgerät 1 oder dem Schweißbrenner 10 beispielsweise über eine Verbindungsleitung 35, wie schematisch mit dem Schweißbrenner 10 dargestellt, oder drahtlos verbunden, wobei für die Übersendung eines Signals an die Sende- und /oder Empfangsvorrichtung 33 die Verbindungsleitung 35 direkt oder indirekt, insbesondere über einen Feldbus, mit der Steuervorrichtung 4 verbunden ist. Bei einer drahtlosen Verbindung der Schutzvorrichtung 28 mit der Schweißanlage oder Stromquelle 2 oder dem Schweißgerät 1, insbesondere mit dem Schweißbrenner 10, ist die Sende- und/oder Empfangsvorrichtung 33 zum Empfang von Funksignalen 36 über elektromagnetische Wellen oder auch optisch, bevorzugt über Infrarotsignale, ausgebildet.

Hierzu weist zumindest eine Komponente, insbesondere des Schweißgerätes 1, eine weitere kompatible Sende- und/oder Empfangsvorrichtung 34, wie im Grundgehäuse 26 strichliert dargestellt, auf, so daß ein entsprechender Datenaustausch bzw. eine Übersendung eines Funksignals 36 bzw. Steuersignals durchgeführt werden kann. Selbstverständlich ist es möglich, daß beide Systeme, also die drahtgebundene und die drahtlose Verbindung, eingesetzt werden können, so daß der Schweißer die Verbindungsart wählen kann.

Damit bei einer drahtlosen Verbindung der Schutzvorrichtung 28 mit dem Schweißgerät 1 die in der Schutzvorrichtung angeordnete Komponente, insbesondere die Sende- und/oder Empfangsvorrichtung 33 und die Ansteuervorrichtung 32, mit Energie versorgt werden können, ist in der Schutzvorrichtung 28 eine Energiequelle 37, insbesondere eine Batterie und/oder eine Solarzelle, angeordnet, wobei für die Ansteuerung des Schutzvisiers 31 mit Energie diese ebenfalls aus der Energiequelle 37 entnommen wird.

Aktiviert ein Benutzer bzw. Schweißer zum Start eines Schweißprozesses den Startschalter 27, so wird ein Signal bzw. Startbefehl an die Steuervorrichtung 4 übersandt, worauf von der Steuervorrichtung 4 die einzelnen Startprozeduren aktiviert werden. Dabei wird zuerst von der Steuervorrichtung 4 das Steuerventil 6 angesteuert, so daß innerhalb einer voreinstellbaren Gasvorlaufzeit eine Schutzgasatmosphäre 38 um den Schweißdraht 13 am Schweißbrenner 10 aufgebaut wird. Gleichzeitig mit dem Start der Gasvorlaufzeit wird von der Steuervorrichtung 4 ein Funksignal 36 bzw. ein Steuersignal über die Sende- und/oder Empfangsvorrichtung 33 und 34 an die Ansteuervorrichtung 32 ausgesendet, wodurch von der Ansteuervorrichtung 32 der Verdunkelungsvorgäng für das Schutzvisier 31 aktiviert wird, d.h., daß von der Ansteuervorrichtung 32 eine Beaufschlagung von Energie an das Schutzvisier 31 eingeleitet wird.

Durch das gleichzeitige Aktivieren der Gasvorlaufzeit und dem Verdunkelungsvorgang für das Schutzvisier 31 wird nunmehr sichergestellt, daß vor dem Zünden des Lichtbogens 15 bereits eine Verdunkelung des Schutzvisiers 31 sichergestellt ist und somit keine Folgeschäden für den Schweißer entstehen können, wie dies bei den Verdunkelungsverfahren aus dem Stand der Technik der Fall ist.

Weiters besteht bei einem derartigen Vorgehen bzw. Steuerverfahren die Möglichkeit, daß die Steuervorrichtung 4 oder direkt vom Schweißbrenner 10 in Abhängigkeit der voreingestellten Schweißparameter, insbesondere eines Schweißstromes, unterschiedliche Signale bzw. Funksignale 36 an die Ansteuervorrichtung 32 übersendet, wobei durch das übersendete Signal die Ansteuervorrichtung 32 den Verdunklungsgrad für das Schutzvisier 31 festlegt, d.h., daß dadurch bei unterschiedlichen Parametern, insbesondere der Höhe des Schweißstromes, eine Anpassung an die entstehende Lichtintensität vorgenommen werden kann und somit der Verdunkelungsgrad für das Schutzvisier 31 geregelt werden kann. Dadurch wird in vorteilhafter Weise erreicht, daß bei einer niedrigen Lichtintensität ein geringerer Verdunkelungsgrad eingestellt wird und somit der Schweißer ohne Sichtprobleme den Schweißprozeß einwandfrei verfolgen kann. Dazu ist es auch möglich, daß in Abhängigkeit der eingestellten Schweißparameter von der Steuervorrichtung 4 die Vorlaufzeit und/oder die Gasvorlaufzeit festgelegt wird, so daß für die Verdunkelung des Schutzvisiers 31 unterschiedliche Zeiten zur Verfügung stehen und somit immer sichergestellt ist, daß bereits vor dem Zünden des Lichtbogens 15 die Verdunkelung des Schutzvisiers 31 abgeschlossen ist.

Nachdem von der Steuervorrichtung 4 die Gasvorlaufzeit und somit der Verdunkelungsprozeß abgelaufen ist, wird von der Steuervorrichtung 4 der Schweißprozeß, also die Zündung des Lichtbogens 15, eingeleitet. Beendet der Schweißer den Schweißprozeß, d.h., daß der Lichtbogen 15 beendet wird, so wird dies von der Steuervorrichtung 4 erkannt, so daß ein neuerliches Signal bzw. Funksignal 36 oder Startbefehl zum Deaktivieren des Schutzvisiers 31 an die Ansteuervorrichtung 32 übersandt wird, worauf von dieser die Energiezufuhr an das Schutzvisier 31 unterbrochen wird und somit die Verdunkelung des Schutzvisiers 31 aufgehoben wird. Dabei ist es jedoch vorteilhaft, daß bei einer Unterbrechung des Lichtbogens 15 von der Steuervorrichtung 4 eine Nachlaufzeit gestartet wird, so daß erst nach Ablauf dieser Nachlaufzeit die Verdunkelung des Schutzvisiers 31 aufgehoben wird. Dadurch wird nämlich erreicht, daß bei einem Kurzschluß zwischen der Elektrode, insbesondere dem Schweißdraht 13, und dem Werkstück 16 der Verdunkelungsgrad nicht aufgehobenwird, so daß bei einer neuerlichen Zündung keine Verdunkelung mehr vorliegt. Dabei ist es auch möglich, daß die Nachlaufzeit als Gasnachlaufzeit verwendet wird und somit die Schutzgasatmosphäre 38 nach Beendigung des Schweißprozesses noch aufrechterhalten bleibt.

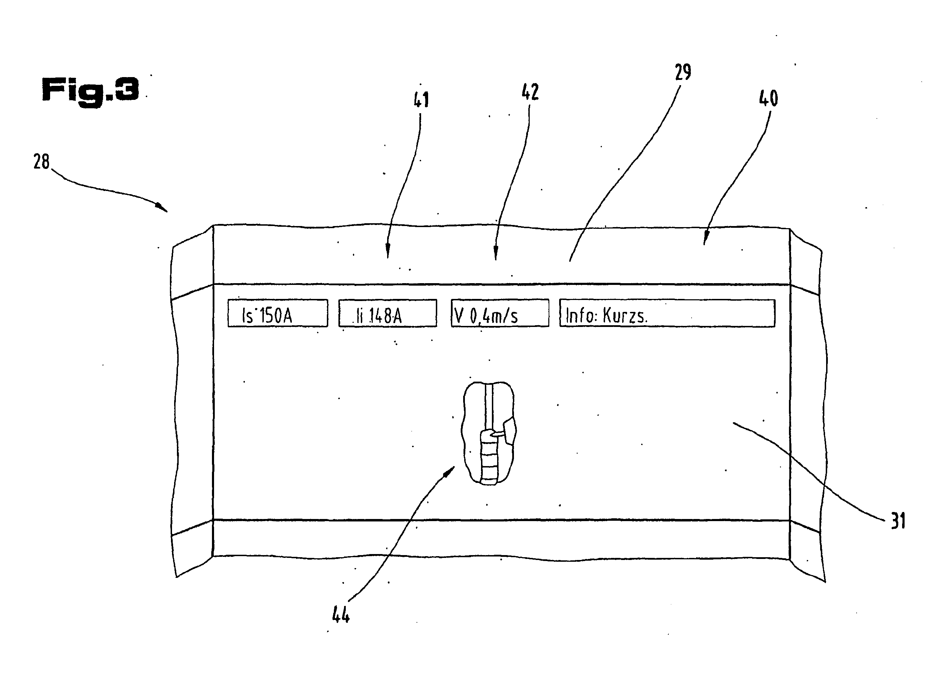

Weiters ist es möglich, daß die Schutzvorrichtung 28, insbesondere der Schutzschirm 29 oder eine an einem Schweißroboter bzw. Schweißautomaten angeordnete Schutzabdeckungen im Schutzschirm 29 und/oder mit dem Traggestell 30 eine Bilderzeugungsvorrichtung 39 angeordnet oder verbunden ist, welche eine Daten- und/oder Bildanzeige 40 von einstellbaren Schweißparametern und/oder Schweißprozeßbilder im Bereich des Schutzvisiers 31 erzeugt, wie dies aus der in der Beschreibungseinleitung abgehandelten DE 40 37 879 A1 bekannt ist. Dazu sind, wie aus Fig. 3 ersichtlich, die Schweißparameter des Schweißstromes 41 und der Drahtvorschubgeschwindigkeit 42 dargestellt. Selbstverständlich ist es möglich, daß mehrere Schweißparameter angezeigt werden können.

Selbstverständlich ist es möglich, daß die Schutzvorrichtung 28 nur mit der Bilderzeugungsvorrichtung 39 und der Energiequelle 37 ausgestattet wird, wobei hierzu wiederum eine drahtlose und/oder drahtgebundene Verbindung mit einer Komponente der Schweißanlage bzw. dem Schweißgerät 1 verbunden sein kann.

Die Bilderzeugungsvorrichtung 39 kann wiederum mit der Sende- und/oder Empfangsvorrichtung 34 und der Energiequelle 37, die aus der Batterie und/ oder aus an dem Schutzvisier 31 angeordneten Solarzellen gebildet ist, oder mit der Ansteuervorrichtung 32 verbunden sein. Das von der Bilderzeugungsvorrichtung 39 erzeugte virtuelle Bild, insbesondere die Daten- und/oder Bildanzeige 40, bildet einem Teilbereich des Schutzvisiers 31 aus, d.h., daß an dem Schutzvisier 31 ein virtuelles Bild eingeblendet wird, welches jedoch derartig ausgebildet ist, daß der Schweißer trotz dieses Bildes den Schweißprozeß erkennen kann.

Das virtuelle Bild ist durch Anzeige von am Schweißgerät 1 bzw. an der Schweißanlage oder einer anderen Eingabevorrichtung festgelegter Schweißparameter gebildet, d.h., daß von der Steuervorrichtung 4 diese Schweißparameter über die Sende- und/oder Empfangsvorrichtungen 33 und 34 an die Schutzvorrichtung 28 übertragen werden und anschließend an die Bilderzeugungsvorrichtung 39 weitergeleitet werden, so daß von dieser ein entsprechendes virtuelles Bild, welches beispielsweise durch die eingestellten Sollwerte und/oder Istwerte der Schweißparameter gebildet ist, dargestellt werden kann. Der Aufbau des virtuellen Bildes kann dabei beliebig erfolgen, da jede aus dem Stand derTechnik bekannte Bilderzeugungsvorrichtung 39 eingesetzt werden kann.

Damit wird nunmehr zusätzlich oder unabhängig von dem zuvor beschriebenen Steuerverfahren ein Verfahren zum Anzeigen von Daten oder Bildern, insbesondere von Schweißparametern, an der Schutzvorrichtung 28, insbesondere dem Schutzschirm 29, bei dem von einem Benutzer bzw. Schweißer über die Ein- und/oder Ausgabevorrichtung 22 an der Schweißanlage oder dem Schweißgerät 1 einzelne Schweißparameter für einen Schweißprozeß eingestellt werden, durchgeführt, wobei von der im Schweißgerät 1 angeordneten Sende- und/oder Empfangsvorrichtung 34 Daten von zumindest einem festgelegten Schweißparameter an die weitere in der Schutzvorrichtung 28, insbesondere dem Schutzschirm 29 oder einer Schutzabdekkung, angeordnete Sende- und/oder Empfangsvorrichtung 33 übersendet werden, die anschließend diese Daten an die Bilderzeugungsvorrichtung 39 übergibt, worauf von dieser die Daten- und/oder Bildanzeige 40 die Schweißparameter und/oder ein Schweißprozeßbild an der Schutzvorrichtung 28 erzeugt. Dabei wird ein kontinuierlicher oder periodischer Datentransfer zwischen der Schweißanlage bzw. dem Schweißgerät 1 und der Schutzvorrichtung 28 bzw. dem Schutzschirm 29 oder umgekehrt durchgeführt, so daß eine Echtzeitanzeige der Sollwerte und/oder Istwerte der Schweißparameter geschaffen wird.

Ebenfalls ist es möglich, daß im Schutzschirm eine Videokamera oder ein anderes optisches Aufnahmegerät 43, wie schematisch dargestellt, angeordnet ist, welches derartig positioniert ist, daß dieses den Sichtbereich des Schutzvisiers 31 vom Benutzer bzw. Schweißers abdeckt und somit ein entsprechendes Bild 44 der Umgebung bzw. des Schweißprozesses in Echtzeit erzeugt und an die Bilderzeugungsvorrichtung 39 weitergeleitet werden kann. Damit kann ein weiteres virtuelles Bild 44 mit den Daten der Videokamera oder des optischen Aufnahmegerätes 43 im Bereich des Schutzvisiers 31 ausbildet werden, so daß beispielsweise das Schutzvisier 31 undurchsichtig ausgebildet werden kann, da über das optische Aufnahmegerät 43 ein entsprechendes Bild 44 erzeugt wird, welches von der virtuellen Bilderzeugungsvorrichtung 39 dem Schweißer sichtbar gemacht wird.

Durch diese Ausbildung der Schutzvorrichtung 28 zum Erzeugen einer virtuellen Daten- und/oder Bildanzeige 40 im Bereich des Schutzvisiers 31 wird in vorteilhafter Weise erreicht, daß bei einem verdunkelten Schutzvisier 31 der Benutzer die wichtigsten Schweißparameter jederzeit erkennen kann und somit die Schweißqualität der gebildeten Schweißraupe erhöht wird, da jederzeit ein Eingriff in den Schweißprozeß durch Erkennen von Abweichungen möglich ist. Dazu ist es selbstverständlich möglich, daß an der Schutzvorrichtung 28 oder am Schweißbrenner 10 Einstellorgane 45 angeordnet sind, so daß eine Änderung der Schweißparameter ohne Unterbrechung des Schweißprozesses möglich ist. Dabei werden die Einstellorgane 45 derartig ausgebildet, daß diese mit den angezeigten Schweißparametern übereinstimmen, d. h., daß beispielsweise am Schweißbrenner 10 zumindest ein Einstellorgan 45 angeordnet ist, welches jeden beliebigen Schweißparameter verstellen kann.

Die Zuordnung dieses Einstellorgans 45 wird dabei von der Steuervorrichtung 4 je nach ängezeigten Schweißparameter am Schutzschirm 29 festgelegt, so daß durch betätigen des Einstellorgans 45 eine Veränderung des Sollwertes des Schweißparameters vorgenommen wird, wobei dieser in Echtzeit an die Bilderzeugungsvorrichtung 39 übertragen wird und somit der Schweißer die vorgenommene Änderung ablesen kann. Selbstverständlich ist es möglich, daß mehrere Einstellorgane 45 angeordnet sein können, oder daß ein Einstellorgan 45 und ein Umschaltelement zur Auswahl der unterschiedlichen Schweißparameter angeordnet ist. Damit wird in vorteilhafter Weise erreicht, daß ein kompakter Aufbau erzielt wird und gleichzeitig eine beliebige Auswahl von Einstellmöglichkeiten geschaffen wird. Wählt der Schweißer dabei einen Schweißparameter, an der zuvor nicht für die Anzeige am Schutzvisier 31 ausgewählt wurde, so wird von der Steuervorrichtung 4 dieser Schweißparameter an die Bilderzeugungsvorrichtung 39 für die Anzeige am Schutzschirm 29 übersandt, so daß der Schweißer nunmehr den neuen Schweißparameter, insbesondere dessen Ist- bzw. Sollwert, und eine für den Schweißparameter hinterlegte Kurzbezeichnung erkennen und ablesen kann.

Weiters ist es möglich, daß die Position der Anzeige für die Schweißparameter vom Benutzer bzw. Schweißerfrei festgelegt wird, d.h., daß, wie es aus dem Stand der Technik bekannt ist, der Schweißer beispielsweise über Einstellorgane am Schutzschirm 29 oder durch einfaches Antasten des Schutzvisiers 31 die Position, wo das virtuelle Bild angezeigt werden soll, festlegen kann und somit beispielsweise von der Bilderzeugungsvorrichtung 39 das virtuelle Bild 44 im Bereich der Augen des Benutzers erzeugt wird, wobei das virtuelle Bild 44 der Daten und/oder Bildanzeige 40 an dem Schweißgerät 1, insbesondere die einzelnen anzuzeigenden Schweißparameter, frei auswählbar sind. Dies ist auch beim Einsatz von der im Schutzschild 31 integrierten Videokamera oder dem optischen Aufnahmegerät 43, von welchem ein kontinuierliches Bild 44, insbesondere ein Schweißbild von dem Schweißprozeß, aufgenommen und an die Bilderzeugungsvorrichtung 39 weitergeleitet wird, worauf von der Bilderzeugungsvorrichtung 39 ein weiteres Bild 44 im Bereich der Augen erzeugt wird, möglich. Selbstverständlich ist es dabei möglich, daß von einer oder mehreren Bilderzeugungsvorrichtungen 39 mehrere virtuelle Bilder an der Schutzvorrichtung 28, insbesondere im Bereich des Schutzvisiers 31, erzeugt werden.

### Bezugszeichenaufstellung

- 1: Schweißgerät
- 2: Stromquelle
- 3: Leistungsteil
- 4: Steuervorrichtung
- 5: Umschaltglied

- 6: Steuerventil
- 7: Versorgungsleitung
- 8: Gas
- 9: Gasspeicher
- 10: Schweißbrenner

- 11: Drahtvorschubgerät
- 12: Versorgungsleitung
- 13: Schweißdraht
- 14: Vorratstrommel
- 15: Lichtbogen

- 16: Werkstück
- 17: Schweißleitung
- 18: Schweißleitung
- 19: Kühlkreislauf
- 20: Strömungswächter

- 21: Wasserbehälter
- 22: Ein- und/oder Ausgabevorrichtung
- 23: Schlauchpaket
- 24: Verbindungsvorrichtung
- 25: Zugentlastungsvorrichtung

- 26: Gehäuse
- 27: Startschalter
- 28: Schutzvorrichtung
- 29: Schutzschirm
- 30: Traggestell

- 31: Schutzvisier
- 32: Ansteuervorrichtung
- 33: Sende- und/oder Empfangsvorrichtung
- 34: Sende- und/oder Empfangsvorrichtung
- 35: Verbindungsleitung

- 36: Funksignal
- 37: Energiequelle
- 38: Schutzgasatmosphäre
- 39: Bilderzeugungsvorrichtung
- 40: Daten- und/oder Bildanzeige

- 41: Schweißstrom
- 42: Drahtvorschubgeschwindigkeit
- 43: Aufnahmegerät
- 44: Bild
- 45: Einstellorgan

## Patentansprüche

1. Schweißanlage mit einem Schweißgerät (1) und einer Schutzvorrichtung (28), insbesondere einem Schutzschirm (29) für einen Benutzer bzw. Schweißer, wobei die Schutzvorrichtung ein elektrisch steuer- und/oder regelbares Schutzvisier (31) mit einer Ansteuervorrichtung (32) aufweist, und das Schweißgerät bzw. die Schweißanlage zumindest durch eine Stromquelle, einen Schweißbrenner (10) und eine Steuervorrichtung (4) gebildet ist, **dadurch gekennzeichnet, daß** die Schutzvorrichtung (28) eine Sende- und/oder Empfangsvorrichtung (33) aufweist, welche mit der Ansteuervorrichtung (32) verbunden ist, wobei die Sende- und/oder Empfangsvorrichtung (33) mit der Schweißanlage oder dem Schweißgerät (1) oder dem Schweißbrenner (10) über eine Verbindungsleitung (35) oder drahtlos *durch eine weitere Sende- und*/*oder Empfangsvorrichtung (34) einer Komponente des Schweißgerätes (1)* verbunden ist und daß die Steuervorrichtung (4) oder der Schweißbrenner (10) bei Aktivieren eines am Schweißbrenner (10) oder am Schweißgerät (1) angeordneten Startschalters (27) oder durch einen Startbefehl ein Signal über die Sende- und/oder Empfangsvorrichtung (33) an die Ansteuervorrichtung (32) zum Verdunkeln des Schutzvisiers (31) aussendet.

2. Schweißanlage nach Anspruch 1, **dadurch gekennzeichnet, daß** die Steuervorrichtung (4) oder der Schweißbrenner (10) in Abhängigkeit der voreingestellten Schweißparameter, insbesondere eines Schweißstromes, unterschiedliche Signale bzw. Funksignale (36) an die Ansteuervorrichtung (32) übersendet, wobei durch das übersendete Signal bzw. das Funksignal (36) die Ansteuervorrichtung (32) den Verdunklungsgrad für das Schutzvisier (31) festlegt.

3. Schweißanlage nach Anspruch 1 oder 2, **dadurch gekennzeichnet, daß** in der Schutzvorrichtung (28) eine Energiequelle (37), insbesondere eine Batterie oder eine Solarzelle, angeordnet ist.

4. Schweißanlage nach einem oder mehreren dervorhergehenden Ansprüche, **dadurch gekennzeichnet, daß** die Steuervorrichtung (4) oder der Schweißbrenner (10) bei Beendigung des Schweißprozesses ein neuerliches Signal bzw. Funksignal oder einen Startbefehl über die Sende- und/oder Empfangsvorrichtung (33) an die Ansteuervorrichtung (32) zum Deaktivieren des Schutzvisiers (31) aussendet.

5. Schweißanlage nach einem oder mehreren der vorhergehenden Ansprüche, **dadurch gekennzeichnet, daß** im Schutzschirm (29) und/oder auf einem Traggestell (30) eine Bilderzeugungsvorrichtung (39) angeordnet oder verbunden ist, welches eine Daten- und/oder Bildanzeige (40) von zumindest einem einstellbaren Schweißparameter und/oder ein Schweißprozeßbild im Bereich des Schutzvisiers (31) erzeugt.

6. Schweißanlage nach Anspruch 5, **dadurch gekennzeichnet, daß** die Bilderzeugungsvorrichtung (39) mit einer Sende- und/oder Empfangsvorrichtung (33) und einer Energiequelle (37) verbunden ist.

7. Schweißanlage nach Anspruch 6, **dadurch gekennzeichnet, daß** die Energiequelle (37) beispielsweise aus einer Batterie und/oder aus an dem Schutzschirm (29) angeordneten Solarzellen gebildet ist.

8. Schweißanlage nach einem oder mehreren dervorhergehenden Ansprüche, **dadurch gekennzeichnet, daß** die Sende- und/oder Empfangsvorrichtung (33) drahtlos oder über eine Verbindungsleitung (34) mit einer weiteren in einem Schweißgerät (1) oder einem Schweißbrenner (10) angeordneten Sende- und/oder Empfangsvorrichtung (36) verbunden ist.

9. Schweißanlage nach einem oder mehreren dervorhergehenden Ansprüche 5 mit 7, **dadurch gekennzeichnet, daß** das von der Bilderzeugungsvorrichtung (39) erzeugte virtuelle Bild (44) bzw. eine Daten- und/oder Bildanzeige (40) einen Teilbereich des Schutzvisiers (31) ausbildet.

10. Schweißanlage nach einem oder mehreren dervorhergehenden Ansprüche 5 mit 7 oder 9, **dadurch gekennzeichnet, daß** das virtuelle Bild (44) der Daten- und/oder Bildanzeige (40) an dem Schweißgerät (1) frei auswählbar ist.

11. Schweißanlage nach einem oder mehreren dervor-hergehenden Ansprüche 5 mit 7 oder 9 mit 10, **dadurch gekennzeichnet, daß** das virtuelle Bild (44) bzw. die Daten und/oder Bildanzeige (40) durch die eingestellten Sollwerte und/oder Istwerte der Schweißparameter gebildet ist.

12. Schweißanlage nach einem oder mehreren der vorhergehenden Ansprüche 5 mit 7 oder 9 mit 11, **dadurch gekennzeichnet, daß** im Schutzschirm (29) eine Videokamera oder ein anderes optisches Aufnahmegerät (43) angeordnet ist, welches derartig positioniert ist, daß dieses den Sichtbereich des Schutzvisiers (31) vom Benutzer abdeckt und ein entsprechendes Bild (44) in Echtzeit erzeugt und an die Bilderzeugungsvorrichtung (39) weiterleitet, welche ein weiteres virtuelles Bild (44) mit den Daten der Videokamera oder des optischen Aufnahmegerätes (43) im Bereich des Schutzvisiers (31) ausbildet.

13. Schweißanlage nach einem oder mehreren der vorhergehenden Ansprüche 5 mit 7 oder 9 mit 12, **dadurch gekennzeichnet, daß** die Schutzvorrichtung (28) derartig ausgebildet ist, daß diese bei einem Schweißroboter als Schutzabdeckung einsetzbar ist.

14. Steuerverfahren für eine Schweißanlage mit einem Schweißgerät (1) und einer Schutzvorrichtung (28), insbesondere einem Schutzschirm (29) für einen Benutzer, bei dem durch Betätigen eines Startschalters (27) an einem Schweißbrenner (10) oder einem Schweißgerät ein Startsignal an eine Steuervorrichtung (4) des Schweißgerätes oder einer Stromquelle zum Aktivieren eines Schweißprozesses erzeugt wird, **dadurch gekennzeichnet, daß** bei der Aktivierung des Startschalters (27) ein Startsignal oder Startbefehl über eine Sende- und/oder Empfangsvorrichtung (33) an eine Ansteuervorrichtung (32) der Schutzvorrichtung (28) übersandt wird, worauf von der Ansteuervorrichtung ein elektrisch steuer- und/oder regelbares Schutzvisier (31) durch Anlegen von Energie aktiviert wird und anschließend der Schweißprozeß, insbesondere die Zündung eines Lichtbogens, gestartet wird.

15. Steuerverfahren nach Anspruch 14, **dadurch gekennzeichnet, daß** der Schweißprozeß, insbesondere die Zündung des Lichtbogens erst nach Ablauf einer voreinstellbaren Vorlaufzeit und/oder einer Gasvorlaufzeit, in der die Verdunkelung des Schutzvisiers erfolgt, gestartet wird.

16. Steuerverfahren nach Anspruch 14 oder 15, **dadurch gekennzeichnet, daß** in Abhängigkeit der eingestellten Schweißparameter von der Steuervorrichtung die Vorlaufzeit und/oder die Gasvorlaufzeit festgelegt wird.

17. Steuerverfahren nach einem oder mehreren der Ansprüche 14 bis 16, **dadurch gekennzeichnet, daß** der Verdunkelungsgrad des Schutzvisiers in Abhängigkeit der eingestellten Schweißparameter festgelegt wird, wobei von der Steuervorrichtung ein entsprechendes Signal an die Schutzvorrichtung übersandt wird.

18. Steuerverfahren nach einem oder mehreren der Ansprüche 14 bis 17, **dadurch gekennzeichnet, daß** bei Beendigung des Schweißprozesses, also beim Erlöschen des Lichtbogens, ein neuerliches Signal bzw. Funksignal oder ein Startbefehl von der Steuervorrichtung ausgesandt wird, worauf von der Ansteuervorrichtung die Energiezufuhr an das Schutzvisier unterbrochen wird.

19. Steuerverfahren nach einem oder mehreren der Ansprüche 14 bis 18, **dadurch gekennzeichnet, daß** von einer in dem Schweißgerät angeordneten Sende- und/oder Empfangsvorrichtung Daten von zumindest einem festgelegten Schweißparameter an eine weitere in einer Schutzvorrichtung, insbesondere einem Schutzschirm oder einer Schutzabdeckung, angeordneten Sende- und/oder Empfangsvorrichtung übersendet werden, die anschließende diese Daten an eine Bilderzeugungsvorrichtung übergibt, worauf von dieser eine Daten- und/ oder Bildanzeige des Schweißparameters und/oder eines Schweißprozeßbildes an der Schutzvorrichtung erzeugt wird

20. Steuerverfahren nach einem oder mehreren der Ansprüche 14 bis 19, **dadurch gekennzeichnet, daß** ein kontinuierlicher oder periodischer Datentransfer zwischen der Schweißanlage bzw. dem Schweißgerät und der Schutzvorrichtung bzw. dem Schutzschirm oder umgekehrt durchgeführt wird.

21. Steuerverfahren nach einem oder mehreren der Ansprüche 19 bis 20, **dadurch gekennzeichnet, daß** vom Benutzer die anzuzeigenden Schweißparameter über die Ein- und/oderAusgabevorrichtung oder über eine weitere Eingabevorrichtung festgelegt werden.

22. Steuerverfahren nach einem oder mehreren der Ansprüche 19 bis 21, **dadurch gekennzeichnet, daß** die Position der Anzeige für die Schweißparameter vom Benutzer frei festgelegt wird.

23. Steuerverfahren nach einem oder mehreren der Ansprüche 19 bis 22, **dadurch gekennzeichnet, daß** von der Bilderzeugungsvorrichtung ein virtuelles Bild im Bereich der Augen des Benutzers erzeugt wird.

24. Steuerverfahren nach einem oder mehreren der Ansprüche 19 bis 23, **dadurch gekennzeichnet, daß** von einer im Schutzschild integrierter Videokamera oder optischen Aufnahmegerät ein kontinuierliches Bild aufgenommen wird, welches an die Bilderzeugungsvorrichtung weitergeleitetwird, worauf von der Bilderzeugungsvorrichtung ein weiteres Bild im Bereich der Augen erzeugt wird.

25. Steuerverfahren nach einem oder mehreren der Ansprüche 19 bis 24, **dadurch gekennzeichnet, daß** von einer oder mehreren Bilderzeugungsvorrichtungen mehrere virtuelle Bilder an der Schutzvorrichtung erzeugt werden.

## Claims

1. Welding system with a welding device (1) and a protective device (28), in particular a protective shield (29) for a user or welder, the protective device having an electrically controllable and/or adjustable protective visor (31) with a control system (32), and the welding device or the welding system comprising at least one current source, a welding torch (10) and a control unit (4), **characterised in that** the protective device (28) has a transmitter and/or receiver unit (33) which is connected to the control system (32), the transmitter and/or receiver unit (33) being connected to the welding system or the welding device (1) or the welding torch (10) via a connecting line (35) or wirelessly via another transmitter and/or receiver unit (34) of a component of the welding device (1) and, when a start switch (27) disposed on the welding torch (10) or on the welding device (1) is activated or when a start command is issued, the control unit (4) or the welding torch (10) sends a signal via the transmitter and/or receiver unit (33) to the control system (32) to darken the protective visor (31).

2. Welding system as claimed in claim 1, **characterised in that** the control unit (4) or the welding torch (10) transmits different signals or radio signals (36) to the control system (32) depending on pre-set welding parameters, in particular for a welding current, and the control system (32) sets the degree of darkening for the protective visor (31) depending on the transmitted signal or the radio signal (36).

3. Welding system as claimed in claim 1 or 2, **characterised in that** an energy source (37), in particular a battery or a solar cell, is provided in the protective device (28).

4. Welding system as claimed in one or more of the preceding claims, **characterised in that** when the welding process is completed, the control unit (4) or the welding torch (10) transmits an updated signal or radio signal or a start command via the transmitter and/or receiver unit (33) to the control system (32) in order to de-activate the protective visor (31).

5. Welding system as claimed in one or more of the preceding claims, **characterised in that** an image-generating device (39) is integrated in the protective shield (29) or connected to a support frame (30), which generates a data and/or image display (40) of at least one adjustable welding parameter and/ or a welding process image in the region of the protective visor (31).

6. Welding system as claimed in claim 5, **characterised in that** the image-generating device (39) is connected to a transmitter and/or receiver unit (33) and an energy source (37).

7. Welding system as claimed in claim 6, **characterised in that** the energy source (37) is provided in the form of a battery and/or solar cells disposed on the protective shield (29).

8. Welding system as claimed in one or more of the preceding claims, **characterised in that** the transmitter and/or receiver unit (33) is connected wire-Iessly or via a connecting line (34) to another transmitter and/or receiver unit (36) disposed in a welding device (1) or a welding torch (10).

9. Welding system as claimed in one or more of the preceding claims 5 in conjunction with 7, **characterised in that** the virtual image (44) or a data and/ or image display (40) generated by the image-generating device (39) form a part region of the protective visor (31).

10. Welding system as claimed in one or more of the preceding claims 5 in conjunction with 7 or 9, **characterised in that** the virtual image (44) of the data and/or image display (40) may be freely selected from the welding device (1).

11. Welding system as claimed in one or more of the preceding claims 5 in conjunction with 7 or 9 in conjunction with 10, **characterised in that** the virtual image (44) or the data and/or image display (40) is based on the set desired values and/or actual values of the welding parameters.

12. Welding system as claimed in one or more of the preceding claims 5 in conjunction with 7 or 9 in conjunction with 11, **characterised in that** a video camera or another optical recording device (43) is provided in the protective shield (29), which is positioned so that it overlaps with the range of vision of the protective visor (31) of the user and generates a corresponding image (44) in real time which is forwarded to the image-generating device (39) which produces another virtual image (44) with the data from the video camera or the optical recording device (43) in the region of the protective visor (31).

13. Welding system as claimed in one or more of the preceding claims 5 in conjunction with 7 or 9 in conjunction with 12, **characterised in that** the protective device (28) is designed so that it may be used with a welding robot as a protective guard.

14. Control method for a welding system with a welding device (1) and a protective device (28), in particular a protective shield (29) for a user or welder, whereby when a start switch (27) on a welding torch (10) or a welding device is activated, a start signal is generated on a control unit (4) of the welding device or a current source in order to activate the welding process, **characterised in that** when the start switch (27) is activated, a start signal or start command is transmitted via a transmitter and/or receiver unit (33) to a control system (32) of the protective device (28), prompting the control system to activate an electrically controllable and/or adjustable protective visor (31) by applying energy thereto and then initiating the welding process, in particular ignition of an arc.

15. Control method as claimed in claim 14, **characterised in that** the welding process, in particular ignition of the arc, is not started until a pre-settable run-in time and/or a gas run-in time has elapsed during which the protective visor is darkened.

16. Control method as claimed in claim 14 or 15, **characterised in that** the run-in time and/orthe gas run-in time is set by the control unit depending on the welding parameter settings.

17. Control method as claimed in one or more of claims 14 to 16, **characterised in that** the degree to which the protective visor is darkened is set depending on the welding parameter settings, a corresponding signal being sent from the control unit to the protective device.

18. Control method as claimed in one or more of claims 14 to 17, **characterised in that** when the welding process is terminated, in other words when the arc is extinguished, a more up-to-date signal or radio signal or a start command is sent from the control unit, whereupon the energy supply to the protective visor is interrupted by the control system.

19. Control method as claimed in one or more of claims 14 to 18, **characterised in that** data pertaining to at least one fixed welding parameter is sent from a transmitter and/or receiver unit disposed in the welding device to another transmitter and/or receiver unit disposed in a protective device, in particular a protective shield or a protective guard, which then transmits these data to an image-generating device, which uses them to generate a data and/or image display of the welding parameter and/or a welding process image on the protective device.

20. Control method as claimed in one or more of claims 14 to 19, **characterised in that** a continuous or periodic data transfer is operated between the welding system or the welding device and the protective device or protective shield or vice versa.

21. Control method as claimed in one or more of claims 19 to 20, **characterised in that** the welding parameters to be displayed are set by the uservia the input and/or output device or via another input device.

22. Control method as claimed in one ormore of claims 19 to 21, **characterised in that** the position of the display for the welding parameters is freely selected by the user.

23. Control method as claimed in one or more of claims 19 to 22, **characterised in that** a virtual image is generated by the image-generating device in the region of the eyes of the user.

24. Control method as claimed in one or more of claims 19 to 23, **characterised in that** a continuous image is recorded by a video camera or optical recording device disposed in the protective shield and forwarded to the image-generating device, as a result of which another image is produced by the image-generating device in the region of the eyes.

25. Control method as claimed in one or more of claims 19 to 24, **characterised in that** several virtual images are produced on the protective device by one or more image-generating devices.

## Revendications

1. Installation de soudage avec un appareil à souder (1) et un dispositif de protection (28), en particulier un casque de protection (29) pour un utilisateur ou soudeur, où le dispositif de protection comporte une visière de protection (31) pouvant être commandée et/ou réglée électriquement avec un dispositif de commande (32), et l'appareil à souder respectivement l'installation de soudage est constituée d'au moins une source de courant, d'un chalumeau (10) et d'un dispositif de commande (4), **caractérisée en ce que** le dispositif de protection (28) comporte un dispositif d'émission et/ou de réception (33) qui est relié au dispositif de commande (32), où le dispositif d'émission et/ou de réception (33) est relié à l'installation de soudage ou à l'appareil à souder (1) ou au chalumeau (10) par une ligne de liaison (35) ou sans fil par un autre dispositif d'émission et/ou de réception (34) d'un composant de l'appareil à souder (1), et **en ce que** le dispositif de commande (4) ou le chalumeau 10, lors de l'activation d'un commutateur de départ (27) disposé au chalumeau (10) ou à l'appareil à souder (1), ou par une commande de départ, émet un signal par le dispositif d'émission et/ou de réception (33) au dispositif de commande (32) pour obscurcir la visière de protection (31).

2. Installation de soudage selon la revendication 1, **caractérisée en ce que** le dispositif de commande (4) ou le chalumeau (10) transmet en fonction des paramètres de soudage préréglés, en particulier d'un courant de soudage, des signaux ou signaux radio (36) différents au dispositif de commande (32), où par le signal respectivement le signal radio (36) transmis, le dispositif de commande (32) fixe le degré d'obscurcissement de la visière de protection (31).

3. Installation de soudage selon la revendication 1 ou 2, **caractérisée en ce qu'**il est disposé dans le dispositif de protection (28) une source d'énergie (37), en particulier une pile ou une pile solaire.

4. Installation de soudage selon l'une ou plusieurs des revendications précédentes, **caractérisée en ce que** le dispositif de commande (4) ou le chalumeau (10), lors de l'achèvement du processus de soudage, émet de nouveau un signal ou signal radio ou une commande de départ par le dispositif d'émission et/ou de réception (33) au dispositif de commande (32) pour désactiver la visière de protection (31).

5. Installation de soudage selon l'une ou plusieurs des revendications précédentes, **caractérisée en ce qu'**il est disposé ou relié dans le casque de protection (29) et/ou sur un châssis de support (30) un dispositif générateur d'images (39) qui produit un affichage de données et/ou d'images (40) d'au moins un paramètre de soudage réglable et/ou d'une image de processus de soudage au voisinage de la visière de protection (31).

6. Installation de soudage selon la revendication 5, **caractérisée en ce que** le dispositif générateur d'images (39) est relié à un dispositif d'émission et/ ou de réception (33) et à une source d'énergie (37).

7. Installation de soudage selon la revendication 6, **caractérisée en ce que** la source d'énergie (37) est formée par exemple par une batterie et/ou des piles solaires disposées au casque de protection (29).

8. Installation de soudage selon l'une ou plusieurs des revendications précédentes, **caractérisée en ce que** le dispositif d'émission et/ou de réception (33) est relié sans fil ou par une ligne de liaison (34) à un autre dispositif d'émission et/ou de réception (36) disposé dans un appareil à souder (1) ou un chalumeau (10).

9. Installation de soudage selon l'une ou plusieurs des revendications précédentes 5 avec 7, **caractérisée en ce que** l'image virtuelle (44) produite par le dispositif générateur d'images (39) ou un affichage de données et/ou d'images (40) constitue une zone partielle de la visière de protection (31).

10. Installation de soudage selon l'une ou plusieurs des revendications précédentes 5 avec 7 ou 9, **caractérisée en ce que** l'image virtuelle (44) de l'affichage de données et/ou d'images (40) peut être sélectionnée librement à l'appareil à souder (1).

11. Installation de soudage selon l'une ou plusieurs des revendications précédentes 5 avec 7 ou 9 avec 10, **caractérisée en ce que** l'image virtuelle (44) ou l'affichage de données et/ou d'images (40) est formée par les valeurs de consigne réglées et/ou les valeurs réelles des paramètres de soudage.

12. Installation de soudage selon l'une ou plusieurs des revendications précédentes 5 avec 7 ou 9 avec 11, **caractérisée en ce qu'**il est disposé dans le casque de protection (29) une caméra vidéo ou un autre appareil de réception optique (43), qui est positionné de telle sorte que celui-ci couvre la zone de vision de la visière de protection (31) de l'utilisateur et produit une image correspondante (44) en temps réel et la transmet au dispositif générateur d'images (39) qui réalise une autre image virtuelle (44) avec les données de la caméra vidéo ou de l'appareil de réception optique (43) au voisinage de la visière de protection (31).

13. Installation de soudage selon l'une ou plusieurs des revendications précédentes 5 avec 7 ou 9 avec 12, **caractérisée en ce que** le dispositif de protection (28) est réalisé de telle sorte qu'il peut être utilisé dans un robot de soudage comme recouvrement protecteur.

14. Procédé de commande d'une installation de soudage avec un appareil à souder (1) et un dispositif de protection (28), en particulier un casque de protection (29) pour un utilisateur, où par l'actionnement d'un commutateur de départ (27) à un chalumeau (10) ou un appareil à souder, un signal de départ est transmis à un dispositif de commande (4) de l'appareil à souder où une source de courant pour activer un processus de soudage, **caractérisé en ce que** lors de l'activation du commutateur de départ (27), un signal de départ ou instruction de départ est transmis par un dispositif d'émission et/ou de réception (33) à un dispositif de commande (32) du dispositif de protection (28), à la suite de quoi, par le dispositif de commande, une visière de protection (31) pouvant être commandée et/ou réglée d'une manière électrique est activée par l'application d'énergie, et ensuite le processus de soudage, en particulier l'allumage d'un arc, est démarré.

15. Procédé de commande selon la revendication 14, **caractérisé en ce que** le processus de soudage, en particulier l'allumage de l'arc, est démarré seulement après l'écoulement d'un temps de préparation préréglable et/ou d'un temps de préparation de gaz, pendant lequel a lieu l'obscurcissement de la visière de protection.

16. Procédé de commande selon la revendication 14 ou 15, **caractérisé en ce qu'**en fonction des paramètres de soudage réglés, par le dispositif de commande, le temps de préparation et/ou le temps de préparation de gaz est fixé.

17. Procédé de commande selon l'une ou plusieurs des revendications 14 à 16, **caractérisé en ce que** le degré d'obscurcissement de la visière de protection est fixé en fonction des paramètres de soudage réglés, où est transmis par le dispositif de commande un signal correspondant au dispositif de protection.

18. Procédé de commande selon l'une ou plusieurs des revendications 14 à 17, **caractérisé en ce qu'**à la fin du processus de soudage, donc lors de l'extinction de l'arc, un nouveau signal respectivement signal radio ou une instruction de commande est émise par le dispositif de commande, à la suite de quoi le dispositif de commande interrompt l'amenée d'énergie à la visière de protection.

19. Procédé de commande selon l'une ou plusieurs des revendications 14 à 18, **caractérisé en ce que** sont transmises par un dispositif d'émission et/ou de réception disposé dans l'appareil à souder des données d'au moins un paramètre de soudage fixé à un autre dispositif d'émission et/ou de réception disposé dans un dispositif de protection, notamment un casque de protection ou un recouvrement de protection, qui transmet ensuite ces données à un dispositif générateur d'images, à la suite de quoi, par celui-ci est produit un affichage de données et/ ou d'images du processus de soudage au dispositif de protection.

20. Procédé de commande selon l'une ou plusieurs des revendications 14 à 19, **caractérisé en ce qu'**on effectue un transfert de données continu ou périodique entre l'installation de soudage ou l'appareil à souder et le dispositif de protection ou le casque de protection ou inversement.

21. Procédé de commande selon l'une ou plusieurs des revendications 19 à 20, **caractérisé en ce que** l'utilisateur détermine les paramètres de soudage à afficher par le dispositif d'entrée et/ou d'émission ou par un autre dispositif d'entrée.

22. Procédé de commande selon l'une ou plusieurs des revendications 19 à 21, **caractérisé en ce que** la position de l'affichage des paramètres de soudage est fixée librement par l'utilisateur.

23. Procédé de commande selon l'une ou plusieurs des revendications 19 à 22, **caractérisé en ce que** le dispositif générateur d'images produit une image virtuelle au voisinage des yeux de l'utilisateur.

24. Procédé de commande selon l'une ou plusieurs des revendications 19 à 23, **caractérisé en ce que** par une caméra vidéo ou un appareil de prise optique intégré dans le casque de protection, une image continue est prise qui est transmise au dispositif générateur d'images, à la suite de quoi est produit par le dispositif générateur d'images une autre image au voisinage des yeux.

25. Procédé de commande selon l'une ou plusieurs des revendications 19 à 24, **caractérisé en ce que** par un ou plusieurs dispositifs générateur d'images, plusieurs images virtuelles sont produites au dispositif de protection.
